# EUROPEAN PATENT SPECIFICATION

(11) **EP 3 333 270 B1**
(45) Date of publication and mention of the grant of the patent: **04.12.2019**
(21) Application number: 17175155.5
(22) Date of filing: 09.06.2017
(51) Int. Cl.: C12Q 1/70

(54) **METHOD FOR DETECTION CPV 2A, 2B, AND 2C AND FOR DISCRIMINATION WILD TYPE FROM VACCINE TYPE**
VERFAHREN ZUM NACHWEIS VON CPV 2A, 2B UND 2C UND ZUR UNTERSCHEIDUNG EINES WILDTYPS VON EINEM IMPFSTOFFTYP
PROCÉDÉ DE DÉTECTION DE CPV2A, 2B ET 2C ET DE DISCRIMINATION DE TYPE SAUVAGE DE TYPE DE VACCIN

(30) Priority: 07.12.2016 US 201615371223
(43) Date of publication of application: 13.06.2018
(73) Proprietor: Credo Biomedical Pte Ltd., Singapore 268802 (SG)
(72) Inventor: WONG, Jr Winston, 221 New Taipei City (TW); KAO, Stephen, Chang-Chi, 221 New Taipei City (TW); Lai, Ying-Ta, 221 New Taipei City (TW); Hung, Ming-Lung, 221 New Taipei City (TW); Wang, Wei-Chen, 221 New Taipei City (TW)
(74) Representative: Becker Kurig Straus

(56) References cited:
- CN-A- 105 463 132
- CN-B- 103 757 139
- US-A1- 2007 042 354
- DECARO N ET AL: "A real-time PCR assay for rapid detection and quantitation of canine parvovirus type 2 in the feces of dogs", VETERINARY MICROBIOLOGY, ELSEVIER BV, NL, vol. 105, no. 1, 5 January 2005 (2005-01-05), pages 19-28, XP027620340, ISSN: 0378-1135 [retrieved on 2005-01-05]
- FULL LENGTH RESEARCH PAPER ESTABLISHMENT AND EVALUATION OF A NOVEL TAQMAN PROBE-BASED REAL-TIME PCR FOR DETECTION OF CANINE PARVOV: "Establishment and evaluation of a novel TaqMan probe-based real-time PCR for detection of Canine Parvovirus", AFRICAN JOURNAL OF MICROBIOLOGY RESEARCH, vol. 6, no. 13, 16 April 2012 (2012-04-16) , pages 3134-3138, XP055393426, DOI: 10.5897/AJMR11.1185
- T. HIRASAWA ET AL: "Differentiation of Wild- and Vaccine-type Canine Parvoviruses by PCR and Restriction-enzyme Analysis", JOURNAL OF VETERINARY MEDICINE. SERIES B - ZENTRALBLATT FUER VETERINAERMEDIZIN. REINE B., vol. 42, no. 1-10, 12 January 1995 (1995-01-12), pages 601-610, XP055393226, DE ISSN: 0931-1793, DOI: 10.1111/j.1439-0450.1995.tb00754.x
- GALI BINGGA ET AL: "High resolution melting curve analysis as a new tool for rapid identification of canine parvovirus type 2 strains", MOLECULAR AND CELLULAR PROBES., vol. 28, no. 5-6, 1 October 2014 (2014-10-01), pages 271-278, XP055393447, GB ISSN: 0890-8508, DOI: 10.1016/j.mcp.2014.08.001
- MARINA GALLO CALDERN ET AL: "Evolution of Canine Parvovirus in Argentina between years 2003 and 2010: CPV2c has become the predominant variant affecting the domestic dog population", VIRUS RESEARCH, AMSTERDAM, NL, vol. 157, no. 1, 17 February 2011 (2011-02-17), pages 106-110, XP028372426, ISSN: 0168-1702, DOI: 10.1016/J.VIRUSRES.2011.02.015 [retrieved on 2011-02-24]
- DECARO N ET AL: "Diagnostic tools based on minor groove binder probe technology for rapid identification of vaccinal and field strains of canine parvovirus type 2b", JOURNAL OF VIROLOGICAL METHODS, ELSEVIER BV, NL, vol. 138, no. 1-2, 1 December 2006 (2006-12-01), pages 10-16, XP027892291, ISSN: 0166-0934 [retrieved on 2006-12-01]
- Namrata Ramdas Hingonekar: "MOLECULAR CHARACTERIZATION OF VP2 GENE OF CANINE PARVO VIRUS FROM VACCINAL STRAINS BY HINGONEKAR NAMRATA RAMDAS", , 1 January 2011 (2011-01-01), pages 1-122, XP055413936, Retrieved from the Internet: URL:http://krishikosh.egranth.ac.in/bitstr eam/1/5810010602/1/NAMRATA RAMDAS.pdf [retrieved on 2017-10-10]
- VASILEIOS NTAFIS ET AL: "Characterization of Canine Parvovirus 2 Variants Circulating in Greece", JOURNAL OF VETERINARY DIAGNOSTIC INVESTIGATION., vol. 22, no. 5, 1 September 2010 (2010-09-01), pages 737-740, XP055413919, US ISSN: 1040-6387, DOI: 10.1177/104063871002200512
- DECARO N ET AL: "A minor groove binder probe real-time PCR assay for discrimination between type 2-based vaccines and field strains of canine parvovirus", JOURNAL OF VIROLOGICAL METHODS, ELSEVIER BV, NL, vol. 136, no. 1-2, 1 September 2006 (2006-09-01), pages 65-70, XP027892359, ISSN: 0166-0934 [retrieved on 2006-09-01]

### SEQUENCE LISTING

<110> Credo Biomedical Pte Ltd.
<120> METHOD FOR DETECTION CPV 2A, 2B, AND 2C AND FOR DISCRIMINATION
   WILD TYPE FROM VACCINE TYPE
<130> 63316 EP
<150> US 15/371223
   <151> 2016-12-07
<160> 34
<170> PatentIn version 3.5
<210> 1
   <211> 1755
   <212> DNA
   <213> canine parvovirus
<400> 1
   atgagtgatg gagcagttca accagacggt ggtcagcctg ctgtcagaaa tgaaagagct      60
   acaggatctg ggaacgggtc tggaggcggg ggtggtggtg gttctggggg tgtggggatt     120
   tctacgggta ctttcaataa tcagacggaa tttaaatttt tggaaaacgg atgggtggaa     180
   atcacagcaa actcaagcag acttgtacat ttaaatatgc cagaaagtga aaattataga     240
   agagtggttg taaataattt ggataaaact gcagttaacg gaaacatggc tttagatgat     300
   actcatgcac aaattgtaac accttggtca ttggttgatg caaatgcttg gggagtttgg     360
   tttaatccag gagattggca actaattgtt aatactatga gtgagttgca tttagttagt     420
   tttgaacaag aaatttttaa tgttgtttta aagactgttt cagaatctgc tactcagcca     480
   ccaactaaag tttataataa tgatttaact gcatcattga tggttgcatt agatagtaat     540
   aatactatgc catttactcc agcagctatg agatctgaga cattgggttt ttatccatgg     600
   aaaccaacca taccaactcc atggagatat tattttcaat gggatagaac attaatacca     660
   tctcatactg gaactagtgg cacaccaaca aatatatacc atggtacaga tccagatgat     720
   gttcaatttt atactattga aaattctgtg ccagtacact tactaagaac aggtgatgaa     780
   tttgctacag gaacattttt ttttgattgt aaaccatgta gactaacaca tacatggcaa     840
   acaaatagag cattgggctt accaccattt ctaaattctt tgcctcaagc tgaaggaggt     900
   actaactttg gttatatagg agttcaacaa gataaaagac gtggtgtaac tcaaatggga     960
   aatacaaact atattactga agctactatt atgagaccag ctgaggttgg ttatagtgca    1020
   ccatattatt cttttgaggc gtctacacaa gggccattta aaacacctat tgcagcagga    1080
   cgggggggag cgcaaacaga tgaaaatcaa gcagcagatg gtgatccaag atatgcattt    1140
   ggtagacaac atggtcaaaa aactaccaca acaggagaaa cacctgagag atttacatat    1200
   atagcacatc aagatacagg aagatatcca gaaggagatt ggattcaaaa tattaacttt    1260
   aaccttcctg taacagatga taatgtattg ctaccaacag atccaattgg aggtaaaaca    1320
   ggaattaact atactaatat atttaatact tatggtcctt taactgcatt aaataatgta    1380
   ccaccagttt atccaaatgg tcaaatttgg gataaagaat ttgatactga cttaaaacca    1440
   agacttcatg taaatgcacc atttgtttgt caaaataatt gccctggtca attatttgta    1500
   aaagttgcgc ctaatttaac aaatgaatat gatcctgatg catctgctaa tatgtcaaga    1560
   attgtaactt actcagattt ttggtggaaa ggtaaattag tatttaaagc taaactaaga    1620
   gcctctcata cttggaatcc aattcaacaa atgagtatta atgtagataa ccaatttaac    1680
   tatgtaccaa gtaatattgg aggtatgaaa attgtctatg aaaaatctca actagcacct    1740
   agaaaattat actaa                                                     1755
<210> 2
   <211> 60
   <212> DNA
   <213> Artificial Sequence
<220>
   <223> primer
<400> 2
   atagcacatc aagatacagg aagatatcca gaaggagatt ggattcaaaa tattaacttt 60
<210> 3
   <211> 29
   <212> DNA
   <213> Artificial Sequence
<220>
   <223> Reverse primer of VP2
<400> 3
   cctccaattg gatctgttgg tagcaatac 29
<210> 4
   <211> 16
   <212> DNA
   <213> Artificial Sequence
<220>
   <223> probe
<400> 4
   gattcaaaat attaac 16
<210> 5
   <211> 18
   <212> DNA
   <213> Artificial Sequence
<220>
   <223> probe
<400> 5
   tggattcaaa atattaac 18
<210> 6
   <211> 18
   <212> DNA
   <213> Artificial Sequence
<220>
   <223> probe
<400> 6
   gattcaaaat attaactt 18
<210> 7
   <211> 36
   <212> DNA
   <213> Artificial Sequence
<220>
   <223> primer
<400> 7
   atgagtgatg gagcagttca accagacggt ggtcag 36
<210> 8
   <211> 24
   <212> DNA
   <213> Artificial Sequence
<220>
   <223> Forward primer of SNP 36
<400> 8
   atgagtgatg gagcagttca acca 24
<210> 9
   <211> 23
   <212> DNA
   <213> Artificial Sequence
<220>
   <223> Forward primer of SNP 36
<400> 9
   tgagtgatgg agcagttcaa cca 23
<210> 10
   <211> 27
   <212> DNA
   <213> Artificial Sequence
<220>
   <223> Forward primer of SNP 36
<400> 10
   gagtgatgga gcagttcaac cagacgg 27
<210> 11
   <211> 31
   <212> DNA
   <213> Artificial Sequence
<220>
   <223> Reverse primer of SNP 36
<400> 11
   gtacccgtag aaatccccac acccccagaa c 31
<210> 12
   <211> 28
   <212> DNA
   <213> Artificial Sequence
<220>
   <223> Reverse primer of SNP 36
<400> 12
   gaaagtaccc gtagaaatcc ccacaccc 28
<210> 13
   <211> 28
   <212> DNA
   <213> Artificial Sequence
<220>
   <223> Reverse primer of SNP 36
<400> 13
   gtacccgtag aaatccccac acccccag 28
<210> 14
   <211> 16
   <212> DNA
   <213> Artificial Sequence
<220>
   <223> SNP 36 probe sequence
<400> 14
   cagcaggctg accacc 16
<210> 15
   <211> 15
   <212> DNA
   <213> Artificial Sequence
<220>
   <223> SNP 36 probe sequence
<400> 15
   cagcaggctg accac 15
<210> 16
   <211> 961
   <212> DNA
   <213> Artificial Sequence
<220>
   <223> primer
<400> 16
   atgagtgatg gagcagttca accagacggt ggtcagcctg ctgtcagaaa tgaaagagct        60
   acaggatctg ggaacgggtc tggaggcggg ggtggtggtg gttctggggg tgtggggatt       120
   tctacgggta ctttcaataa tcagacggaa tttaaatttt tggaaaacgg atgggtggaa       180
   atcacagcaa actcaagcag acttgtacat ttaaatatgc cagaaagtga aaattataga       240
   agagtggttg taaataattt ggataaaact gcagttaacg gaaacatggc tttagatgat       300
   actcatgcac aaattgtaac accttggtca ttggttgatg caaatgcttg gggagtttgg       360
   tttaatccag gagattggca actaattgtt aatactatga gtgagttgca tttagttagt       420
   tttgaacaag aaatttttaa tgttgtttta aagactgttt cagaatctgc tactcagcca       480
   ccaactaaag tttataataa tgatttaact gcatcattga tggttgcatt agatagtaat       540
   aatactatgc catttactcc agcagctatg agatctgaga cattgggttt ttatccatgg       600
   aaaccaacca taccaactcc atggagatat tattttcaat gggatagaac attaatacct       660
   ctcatactgg aactagtggc acaccaacaa atatatacca tggtacagat ccagatgatg       720
   ttcaatttta tactattgaa aattctgtgc cagtacactt actaagaaca ggtgatgaat       780
   ttgctacagg aacatttttt tttgattgta aaccatgtag actaacacat acatggcaaa       840
   caaatagagc attgggctta ccaccatttc taaattcttt gcctcaagct gaaggaggta       900
   ctaactttgg ttatatagga gttcaacaag ataaaagacg tggtgtaact caaatgggaa       960
   a                                                                       961
<210> 17
   <211> 30
   <212> DNA
   <213> Artificial Sequence
<220>
   <223> Forward primer
<400> 17
   caaacaaata gagcattggg cttaccacca 30
<210> 18
   <211> 32
   <212> DNA
   <213> Artificial Sequence
<220>
   <223> forward primer
<400> 18
   ggcttaccac catttctaaa ttctttgcct ca 32
<210> 19
   <211> 30
   <212> DNA
   <213> Artificial Sequence
<220>
   <223> forward primer
<400> 19
   caaacaaata gagcattggg cttaccacca 30
<210> 20
   <211> 30
   <212> DNA
   <213> Artificial Sequence
<220>
   <223> forward primer
<400> 20
   tctttgcctc aagctgaagg aggtactaac 30
<210> 21
   <211> 26
   <212> DNA
   <213> Artificial Sequence
<220>
   <223> forward primer
<400> 21
   tctttgcctc aagctgaagg aggtac 26
<210> 22
   <211> 30
   <212> DNA
   <213> Artificial Sequence
<220>
   <223> Reverse primer
<400> 22
   gcactataac caacctcagc tggtctcata 30
<210> 23
   <211> 30
   <212> DNA
   <213> Artificial Sequence
<220>
   <223> Reverse primer
<400> 23
   acaccacgtc ttttatcttg ttgaactcct 30
<210> 24
   <211> 20
   <212> DNA
   <213> Artificial Sequence
<220>
   <223> probe
<400> 24
   ctgaaggagg tactaacttt 20
<210> 25
   <211> 20
   <212> DNA
   <213> Artificial Sequence
<220>
   <223> probe for SNP 899
<400> 25
   ctgaaggagg tactaacttt 20
<210> 26
   <211> 20
   <212> DNA
   <213> Artificial Sequence
<220>
   <223> probe for SNP 899
<400> 26
   tgaaggaggt actaactttg 20
<210> 27
   <211> 21
   <212> DNA
   <213> Artificial Sequence
<220>
   <223> probe for SNP 899
<400> 27
   ctgaaggagg tactaacttt g 21
<210> 28
   <211> 24
   <212> DNA
   <213> Artificial Sequence
<220>
   <223> probe for SNP 899
<400> 28
   agctgaagga ggtactaact ttgg 24
<210> 29
   <211> 23
   <212> DNA
   <213> Artificial Sequence
<220>
   <223> Probe for SNP 963
<400> 29
   tcaaatggga aatacaaact ata 23
<210> 30
   <211> 21
   <212> DNA
   <213> Artificial Sequence
<220>
   <223> Probe for SNP 963
<400> 30
   caaatgggaa atacaaacta t 21
<210> 31
   <211> 23
   <212> DNA
   <213> Artificial Sequence
<220>
   <223> Probe for SNP 963
<400> 31
   tcaaatggga aatacaaact ata 23
<210> 32
   <211> 23
   <212> DNA
   <213> Artificial Sequence
<220>
   <223> Probe for SNP 963
<400> 32
   tcaaatggga aatacaaact ata 23
<210> 33
   <211> 24
   <212> DNA
   <213> Artificial Sequence
<220>
   <223> Probe for SNP 963
<400> 33
   actcaaatgg gaaatacaaa ctat 24
<210> 34
   <211> 28
   <212> DNA
   <213> Artificial Sequence
<220>
   <223> Probe for SNP 963
<400> 34
   ctaccacaac aggagaaaca cctgagag 28

## Description

This disclosure is related to a method according to the pre-characterizing clauses of claims 1, 6 and 13.

Diagnosis of CPV-2a, CPV-2b, and CPV-2c may be ambiguous when carried out on fecal samples from dogs presenting with diarrhea few days after vaccination. In fact, the modified-live virus contained in the vaccines is able to replicate in the intestinal mucosa of vaccinated dogs, despite the unnatural route of administration, and to be shed in the feces albeit at low titers and for a shorter time period with respect to the wild strands. In such a circumstance, the detection of the nucleic acid of CPV-2a, 2b, and 2c in the feces of vaccinated dogs could be false-positive, leading to a misdiagnosis of the infection.

In some situation, the CPV vaccinal strains may be detected by PCR, cloning, sequencing, sequence analysis with available sequences in GenBank followed by phologenetic analysis. However, sequences of the VP2 gene of CPV from vaccine strains are different from each other, as compared to reference strains, so that, the diagnosis of CPV may take lots steps to achieve and also be time-consuming (*see*, Namrate Ramdas Hingonekar: MOLECULAR CHARACTERIZATION OF VP2 GENE OF CANINE PARVO VIRUS FROM VACCINAL STRAINS BY HINGONEKAR NAMRATA RAMDAS; January 2011, pages 1-122) (available at: http://krishikosh.egranth.ac.in/displaybitstream?handle=1/5810010602) .

Recently, real time PCR assays have been established for identification of type 2a, 2b and 2c using TaqMan probes with conjugated minor groove binder (MGB) ligands. In such assay, primers and MGB probes specific for the type 2b vaccines and field strains were designed and used, with the MGB probe being able to discriminate between vaccine strain SAH and field strains of CPV-2b by taking account of SNP T3749A, SNP T3575G, or other variations in the VP2 gene (*see,* Decaro N et al.: Diagnostic tools based on minor groove binder probe technology for rapid identification of vaccinal and field strains of canine parvovirus type 2b; Journal of virological methods, Elsevier BV, NL vol. 138, no. 1-2, 2006, pages 10-16) .

This in mind, the present disclosure aims at providing a method of discriminating a target nucleic acid of wild type from vaccine type in a sample that overcomes the false-positive and the misdiagnosis of the infection and gain accuracy and stability for detection CPV-2a, CPV-2b and CPV-2c and for discriminating wild type from vaccine type.

This in mind, the present disclosure further aims at providing [[a]]another method of discriminating a target nucleic acid of wild type from vaccine type in a sample that overcomes the false-positive and the misdiagnosis of the infection and gain accuracy and stability for detection CPV-2a, CPV-2b and CPV-2c and for discriminating wild type from vaccine type.

This achieved by a method of discriminating a target nucleic acid according to claim 1. The dependent claims pertain to corresponding further developments and improvements.

This also achieved by a method of discriminating a target nucleic acid according to claim 7. The dependent claims pertain to corresponding further developments and improvements.

As will be seen more clearly from the detailed description following below, the claimed method of discriminating a target nucleic acid comprising steps:
(a) providing a sample suspected to contain the target nucleic acid;
(b) providing a pair of primers comprising a first and a second primer characterised in that the first primer consists of at least contiguous 12 nucleotides of a nucleic acid sequence selected from the nucleic acid sequence SEQ ID:7, and characterised in that the second primer consists of at least contiguous 12 nucleotides selected from the complementary nucleic acid sequences SEQ ID NO:11,12, or 13;
(c) amplifying the target nucleic acid with a template-dependent polymerase;
(d) annealing a SNP probe to the target nucleic acid to form a hybridized product during step (c) characterised in that the SNP probe is specific to SNP 36 of the VP2 gene of CPV 2a, 2b, or 2c, and characterised in that the SNP 36 probe sequence is SEQ ID NO:14 or 15, wherein the VP 2 gene is according to SEQ ID NO:1; and
(e) detecting the signals generating from the hybridized product as an indicator of the presence of the target nucleic acid, wherein the presence of the signals is indicative of wild type, and the vaccine type is the sample had once vaccinated with one of the vaccine group including Duramune MX5, Canivac 5, Vanguarad plus 5 L4 CV, Nobivac Puppy DP, Canine 6II-SL, Eurican5, Virbagen DA2Parv.

As will be seen more clearly from the detailed description following below, the claimed method of discriminating a target nucleic acid of wild type from vaccine type in a sample comprising steps:
(f) providing a sample suspected to contain VP2;
(g) providing a pair of primers comprising a first and a second primer characterised in that the first primer consists of at least contiguous 12 nucleotides of a nucleic acid sequence selected from the nucleic acid sequence SEQ ID:16, and characterised in that the second primer consists of at least contiguous 12 nucleotides selected from the complementary nucleic acid sequences SEQ ID NO: 22 or 23;
(h) amplifying the sample with a template-dependent polymerase;
(i) annealing both P1 and a P2 probes to the target nucleic acid to form a hybridized product during step (h) characterised in that the P1 probe is specific to SNP 899 of the VP2 gene of CPV 2a, 2b, or 2c and the P2 probe is specific to SNP 963 of the VP2 gene of CPV 2a, 2b, or 2c, characterised in that the P1 is selected from a group consisting of SEQ ID NO: 24 to 27, and the P2 is selected from a group consisting of SEQ ID NO:29 to 33, wherein the VP 2 gene is according to SEQ ID NO:1; and
(j) detecting two distinct fluorescent signals generating from the hybridized product, wherein the presence of both distinct fluorescent signals is indicative of the wild type, the presence of any one of the distinct fluorescent signals is indicative of the vaccine type, and the vaccine type is that the sample had once vaccinated with one of the vaccine group including Duramune MX5, Canivac 5, Vanguarad plus 5 L4 CV, Nobivac Puppy DP, Canine 6II-SL, Eurican5, Virbagen DA2Parvo.

In the following, the disclosure is further illustrated by way of example, taking reference to the accompanying drawings. Thereof
FIG.1 is the amplification products derived from the aspect 1 by means of agarose gel electrophoresis.
FIG. 2 is the kinetic PCR growth curves of aspect 1.
FIG.3 is the amplification products derived from the aspect 2 by means of agarose gel electrophoresis. No.1-4 are wild type samples, and No.5-7 are vaccine type samples. NTC represents the negative control.
FIG. 4 is the kinetic PCR growth curves of aspect 2. No.1-4 are wild type samples, and No.5-7 are vaccine type samples. NTC represents the negative control.
FIG. 5 is the kinetic PCR growth curves of aspect 2. No.1-4 are authentic specimens, and No.5-6 are from vaccine bulks. NTC represents the negative control.
FIG.6 is the kinetic PCR growth curves of the combination of SNP 899 and SNP963 of aspect 3. 20 authentic specimens are all positive on both SNP 899 and SNP963.
FIG.7 is the kinetic PCR growth curves of the combination of SNP 899 and SNP963 of aspect 3. Vanguard bulk is only positive on SNP 963, and Duramune bulk is only positive on SNP 899.
FIG.8 is the amplification products derived from the aspect 4 by means of agarose gel electrophoresis.
FIG.9 is the lateral flow of aspect 4.
FIG.10 is the amplification products derived from the aspect 5 by means of agarose gel electrophoresis.
FIG.11 is the lateral flow of aspect 5.

Unless otherwise explicitly specified herein, the drawings are not drawn to scale.

In order to solve the questions mentioned above, the present disclosure provides a PCR or real-time PCR assay for rapid identification of CPV 2a, 2b, and 2c using TaqMan probes with conjugated minor groove binder (MGB) ligands.

In such assays, labeling the type-specific probes with different fluorescent reporter has ensured the detection of type-specific fluorescence. The Taq polymerase applied in this assay is a DNA-dependent polymerase with nexonuclease hydrolysis function. The fluorescent signals are detected by the quantity of the fragment of the fluorescent reporter which are cleaved from the probe hybridized to the target nucleic acid by an exonuclease hydrolysis of the DNA-dependent polymerase. The primer and/or probe comprise(s) a modified nucleotide or a non-nucleotide compound.

In one aspect of the present disclosure, a method is provided for the detection of a target nucleic acid comprising the nucleic acid sequence of CPV 2a, 2b, and 2c in a sample comprising the step of:
(a) To provide a sample suspected to contain the target nucleic acid of VP2 of CPV type 2a, 2b, and 2c.
(b) To provide a pair of primers comprising a forward and a reverse primer which the forward primer consists of at least contiguous 12 nucleotides selected from the nucleic acid sequence SEQ ID: 2 of VP2 gene, and the reverse primer consists of at least contiguous 12 nucleotides selected from the complementary nucleic acid sequence SEQ ID NO: 3.
(c) To amplify the target nucleic acid with a template-dependent polymerase.
(d) To anneal the probe to the target nucleic acid to form a hybridized product during step (c) wherein the probe sequence is selected from a group comprising SEQ ID NO:4 to 6, and
(e) To detect signals generating from the hybridized product as an indicator of the presence of the target nucleic acid of the CPV type 2a, 2b, and 2c.

Due to VP2 (SEQ ID: 1) is the conserved region of CPV 2a, 2b, and 2c, all primers and probes disclosed in the present disclosure are selected from VP2 gene. To be more precisely, the forward primer for detection of CPV 2a, 2b, and 2c are consisted of at least contiguous 12 nucleotides of a nucleic acid sequence selected from the nucleic acid sequence SEQ ID: 2, which is part of the VP2 gene, and the reverse primer consists of at least contiguous 12 nucleotides selected from the complementary nucleic acid sequence SEQ ID NO: 3. The probe, which is one of the group of SEQ ID NO: 4 to 6, is also specific to VP2 gene. The presence of the fluorescence signal is indicative CPV 2a, 2b, or 2c in a sample.

The present disclosure also discloses a method for discriminating the wild type from the vaccine by detecting SNP 36, SNP 899 and SNP 963 of VP2 gene. Regarding to SNP 36, the nucleotide is G for wild type and A for vaccine type, where the vaccine type means the sample had once vaccinated with one of the vaccine group including Duramune MX5, Canivac 5, Vanguarad plus 5 L4 CV, Nobivac Puppy DP, Canine 6II-SL, Eurican5, Virbagen DA2Parvo. And for SNP 899, the nucleotide is G for wild type and Duramune MX5 and C for vaccine type, where the vaccine type means the sample had once vaccinated with one of the vaccine group including Canivac 5, Vanguarad plus 5 L4 CV, Nobivac Puppy DP, Canine 6II-SL, Eurican5, Virbagen DA2Parvo. Lastly, in regard to SNP 963, the nucleotide is T for wild type, Canivac 5, Vanguarad plus 5 L4 CV, Nobivac Puppy DP, Canine 6II-SL, Eurican5, Virbagen and DA2Parvo, and A for vaccine type, where the vaccine type means the sample had once vaccinated with Duramune MX5. The TICD50 of the CPV of above-mentioned vaccines are greater or equal to 10⁵ copy. Therefore, the primers and probes are designed specific for the wild type of the present disclosure.

**Table 1. The nucleotide of wild type and vaccine type**

| Sample | SNP 36 | SNP 899 | SNP 963 |
|---|---|---|---|
| Wild type | G | G | T |
| Duramune MX5 | A | G | A |
| Canivac 5 | A | C | T |
| Vanguarad plus 5 L4 CV | A | C | T |
| Nobivac Puppy DP | A | C | T |
| Canine 6II-SL | A | C | T |
| Eurican5 | A | C | T |
| Virbagen DA2Parvo | A | C | T |

In another aspect of the present disclosure, a method is provided for the discrimination wild type from vaccine type by SNP 36 comprising the step of:
(a) providing a sample suspected to contain the target nucleic acid,
(b) providing a pair of primers comprising a forward and a reverse primer wherein the forward primer consists of at least contiguous 12 nucleotides nucleic selected from the nucleic acid sequence SEQ ID:7, and the reverse primer consists of at least contiguous 12 nucleotides selected from the complementary nucleic acid sequences SEQ ID NO:11,12, or 13,
(c) amplifying the subject with a template-dependent polymerase,
(d) annealing a SNP probe to the target nucleic acid to form a hybridized product during step (c), the SNP probe is specific to SNP 36 of the VP2 gene, and the SNP 36 probe sequence is SEQ ID NO:14 or 15,
(e) detecting the signals generating from the hybridized product as an indicator of the presence of the target nucleic acid.

SNP 36 can be applied solely to discriminate the wild type from the vaccine type. The forward primer of SNP 36 is consisted of at least contiguous 12 nucleotides of a nucleic acid sequence selected from the nucleic acid sequence SEQ ID: 7, or is selected one from the group of SEQ ID: 8 to 10. The reverse primer is selected one from the group of SEQ ID: 11 to 13. The probe is selected one from the group of SEQ ID: 14 to 15. The probe also carries a fluorescent reporter which is selected from the group comprising FAM, HEX, VIC, CY5, or TET, and the 3'-terminal of the probe carries a quencher which is selected from a group comprising TMARA, MGB, or BHQ. The presence of the fluorescent signal is indicative of wild type, and the absence of the fluorescent signal is indicative of the vaccine type. The vaccine type means the dog had once vaccinated with one of the vaccine group including Duramune MX5, Canivac 5, Vanguarad plus 5 L4 CV, Nobivac Puppy DP, Canine 6II-SL, Eurican5, Virbagen DA2Parvo. The presence of the fluorescent signal is indicative the wild type, and the absence of fluorescent signal is indicative of the vaccine type.

In still another aspect of the present disclosure, a method is provided for the discrimination wild type from vaccine type by the combination of SNP 899 and SNP 963 comprising the step of:
(f) providing a subject suspected to contain VP2,
(g) providing a pair of primers comprising a forward and a reverse primer wherein the forward primer consists of at least contiguous 12 nucleotides selected from the nucleic acid sequence SEQ ID: 16, and wherein the reverse primer consists of at least contiguous 12 nucleotides selected from the complementary nucleic acid sequences SEQ ID NO: 22 or 23,
(h) amplifying the subject with a template-dependent polymerase,
(i) annealing two probes to the target nucleic acid to form a hybridized product during step (c) that one of the two probe is specific to SNP 899 of the VP2 gene, and the other probe is specific to SNP 963 of the VP2 gene, one probe is selected from a group consisting of SEQ ID NO: 24 to 27, and the other is selected from a group consisting of SEQ ID NO:29 to 33,
(j) detecting two distinct fluorescent signals generating from the hybridized product.

The forward primer is consisted of at least contiguous 12 nucleotides selected from the nucleic acid sequence SEQ ID: 16, or is selected one from the group of SEQ ID: 17 to 21. The reverse primer is selected one from the group of SEQ ID: 22 to 23. The 5'-terminal of two probes also carry distinct fluorescent reporters which are selected from the group comprising FAM, HEX, VIC, CY5, or TET, and the 3'-terminal of the probes carry a quencher which are selected from a group comprising TMARA, MGB, or BHQ.

The probe of SNP 899 is selected one from the group of SEQ ID: 24 to 28, and the probe of SNP 963 is selected one from the group of SEQ ID: 29 to 33. The presence of the both fluorescent signal is indicative of wild type, and the presence of any one of the fluorescent signal is indicative of the vaccine type. The vaccine type means the dog had once vaccinated with one of the vaccine group including Duramune MX5, Canivac 5, Vanguarad plus 5 L4 CV, Nobivac Puppy DP, Canine 6II-SL, Eurican5, Virbagen DA2Parvo. The presence of both distinct fluorescent signals is indicative of the wild type, and the presence of any one of the distinct fluorescent signals is indicative of the vaccine type. The absence of both fluorescent signals is indicative of non-infected or low viral load sample.

The present disclosure using TaqMan probes is more sensitive than traditional method, where the limit of detection (hereinafter LOD) of present disclosure could reach 10¹ copy on both CPV 2a, 2b, 2c detection and wild type/ vaccine type discrimination. Furthermore, the specificity of CPV 2a, 2b, 2c detection and wild type/ vaccine type discrimination is relatively high because the discrimination window could reach 10⁸ copy. Therefore, the present disclosure is suitable for rapid and unambiguous detection of CPV 2a, 2b, and 2c and discrimination wild type from vaccine type.

The present disclosure also provides a lateral flow immunochromatographic assay for rapid identification of CPV 2a, 2b, and 2c. The test results can be observed visually by the colored particles. In such assays, labeling the type-specific probes with different antigen has ensured the detection of type-specific analyte. The primer and/or probe c5'-terminal of either the forward or reverse primer carries an antigen which is selected from a group comprising FITC, DIG, Biotin, Texas-red and Tamra, and the 3'-terminal of the probe carries a distinct antigen from either the first or second primer which is selected from the group comprising FITC, DIG, Biotin, Texas-red and Tamra. The colored particle is selected from a group of colloidal gold, latex, and carbon nanoparticles.

The sequences of both primers and probes, the detection steps, and the amplifying conditions are identical to the Taqman probe detection method of CPV 2a, 2b, and 2c detection except the amount of forward primer is less than reverse primer, and the PNA is added and participated the amplifying step.

After the amplifying step is done, the analyte is added on the stripe to initiate the immunochromatographic assay. The presence of analyte signals in the test-line is indicative of the presence of the CPV 2a, 2b, and 2c in a sample, and the absence of analyte signals in the test-line is indicative of the absence of CPV 2a, 2b, and 2c.

Moreover, the present disclosure also provides a lateral flow immunochromatographic assay for discrimination wild type from vaccine type. The sequences of both primers and probes, the detection steps, and the amplifying conditions are identical to the SNP 36 detection except the amount of forward primer is less than reverse primer, and the PNA is added and participated the amplifying step. After the amplifying step is done, the analyte is added on the stripe to initiate the immunochromatographic assay. The presence of analyte signals in the test-line is indicative of the presence of the wild type, and the absence of analyte signals in the test-line is indicative of the vaccine type.

The present disclosure using lateral flow immunochromatographic assay is sensitive than traditional method, where the detection limit of present disclosure could reach 10¹ copy on both CPV 2a, 2b, 2c detection and wild type/ vaccine type discrimination. Besides, the present disclosure is also specific because it can still detect the CPV 2a, 2b, 2c, or SNP 36 while the vaccine titer is relatively high (10³ copy). Therefore, the present disclosure using lateral flow immunochromatographic assay is suitable for rapid and unambiguous detection of CPV 2a, 2b, and 2c and discrimination wild type from vaccine type.

### ASPECT 1 CPV 2a, 2b, and 2c detection by Taqman probe:

Preferably, the present disclosure does not comprise the step of sample preparation. After purification or isolation of the nucleic acids including the target nucleic acid from a suspected sample, the target nucleic acid may be detected with different conditions.

One CPV2a infected sample, one CPV2b infected sample, one CPV2c infected sample, and one healthy sample are used in aspect 1. The DNA was isolated by using an AXYGEN®AxyPrep Body Fluid Viral DNA/RNA. Primers having SEQ ID NO: 34 and SEQ ID NO: 3 were used to amplify a VP2 2a, 2b, and 2c sequence. The primer and probe sequences are shown in Table 2. The probe can also be replaced to SEQ ID No: 5 or SEQ ID No: 6.

**Table 2. Primer Sequence**

| **Primers used in the examples** | | |
|---|---|---|
| **Sequence ID** | Function | Sequence 5'-3' |
| **SEQ ID No:34** | Forward primer of VP2 | |
| **SEQ ID No:3** | Reverse primer of VP2 | |
| **SEQ ID No:4** | probe | GATTCAAAATATTAAC |

The amplification reaction is carried out which was measured and monitored in real-time on a BioRad CFX Connect Real-time System (BioRad Laboratories, Inc.). Each reaction mixture volume was 20 µl, and was amplified under the following conditions:

**Table 3. Conditions of the Amplification of the reference samples**

| | 2a | 2b | 2c | Healthy |
|---|---|---|---|---|
| DNA template | | 1 µl | | |
| Primer (F) | | 0.5 µM | | |
| Primer (R) | | 0.5 µM | | |
| Probe | | 0.2µM | | |
| dNTP | | 0.25 mM | | |
| Polymerase | | 5 unit | | |
| Total Volume | | 20 µl | | |

The reaction mixtures were firstly incubated for 1 minutes at 95°C. The actual amplification reaction was carried out for 50 cycles according to the following scheme:
95 °C 1sec. → 65 °C 1 sec.

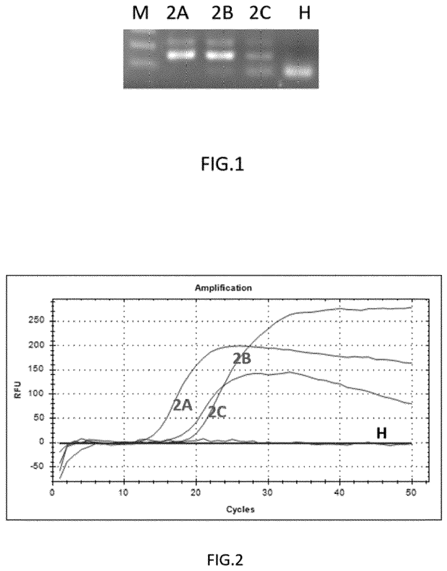

FIG.1 shows the PCR products of the corresponding region, that is VP2, by means of agarose gel. The "H" represents healthy sample, and the "M" represents marker. It demonstrates that the PCR reactions with the given primer do exhibit none or less cross reactivity or amplification of unspecific sequences on both wild type samples and vaccine type samples.

FIG. 2 shows the kinetic PCR growth curve for the given pair of primers and probe. When the growth curves of CPV2a, CPV2b, and CPV2c exceed the threshold, an unambiguous and specific signal is initially detectable. In other words, if the sample contains a VP2 sequence, a climbing curve will show in the kinetic PCR growth curve. In the meantime, no signal is detectable of healthy sample because the primer and probe are not specific to the sequence of the healthy sample.

### ASPECT 2 Discriminating Wild Type from Vaccine Type by Using SNP 36 by Taqman probe:

### A. Specificity and Sensitivity test

In order to verify the specificity and the sensitivity for discriminating wild type from vaccine type by using SNP 36, sequences with known copy number are needed to perform the test. The sequences are prepared by the following steps: 1. Clone the wild type and vaccine type of SNP 36 corresponding region into a designated vector respectively. 2. Transform these two factor into E.coli. 3. Extract the plasmid DNA. 4. Clarify the sequences are correct with PCR or sequencing.

Four serial single dilutions of the wild type of the SNP 36 of VP2 (hereinafter wild type samples) are prepared with copy number of 10⁴, 10³, 10², 10¹, and three serial single dilutions of the vaccine type of the SNP 36 of VP2 (hereinafter vaccine type samples) are prepared with copy number 10⁸, 10⁷, 10⁶.

Primers having SEQ ID NO: 8 for the forward primer and SEQ ID NO: 11 for the reverse primer, and probes having SEQ ID NO: 14 were used to amplify the above-mentioned wild type samples and vaccine type samples. The primer and probe sequences are shown in Table 4.

The forward primers can be replaced to SEQ ID No: 9 or 10, the reverse primers can be replaced to SEQ ID No: 12 or 13, and the probes can be replaced to SEQ ID No: 15. The amplification conditions are identical with the aspect 1.

**Table 4. Primer Sequence**

| **Primers used in the examples** | | |
|---|---|---|
| **Sequence ID** | Function | Sequence 5'-3' |
| **SEQ ID No:8** | Forward primer of SNP 36 | |
| **SEQ ID No:11** | Reverse primer of SNP 36 | |
| **SEQ ID No:14** | probe | CAGCAGGCTGACCACC |

**Table 5. Conditions of the Amplification of SNP 36**

| | Wild type samples | | | | Vaccine type samples | | | |
|---|---|---|---|---|---|---|---|---|
| Sample no. | 1 | 2 | 3 | 4 | 5 | 6 | 7 | NTC |
| Copy no | 10⁴ | 10³ | 10² | 10² | 10⁸ | 10⁷ | 10⁶ | 0 |
| DNA template | | | | | 1 µl | | | |
| Primer (F) | | | | | 0.5 µM | | | |
| Primer (R) | | | | | 0.5 µM | | | |
| Probe | | | | | 0.2µM | | | |
| dNTP | | | | | 0.25 mM | | | |
| Polymerase | | | | | 5 unit | | | |
| Total | | | | | 20 µl | | | |
| Volume | | | | | | | | |

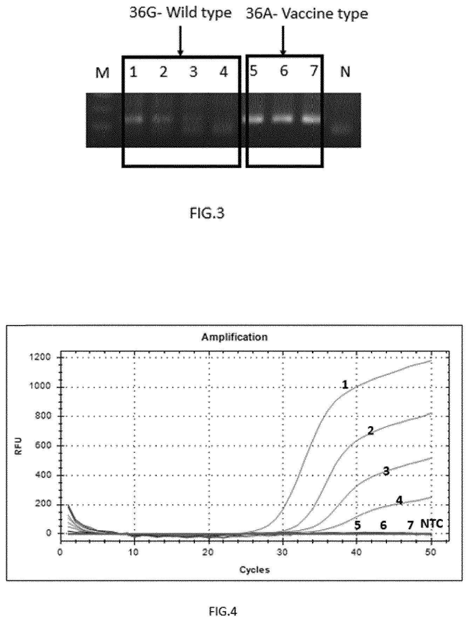

FIG.3 shows the PCR products of the corresponding region, that is SNP 36 of VP2, by means of agarose gel. The "NTC" represents negative control, and the "M" represents marker. It demonstrates that the PCR reactions with the given primer do exhibit none or less cross reactivity or amplification of unspecific sequences on both wild type samples and vaccine type samples.

FIG. 4 shows the kinetic PCR growth curve for the given pair of primers and probe. The probe given in this aspect is specific to wild type of SNP 36. As above-mentioned, the probe is complementary to "G" since the nucleotide is G for wild type. Therefore, when the growth curve of sample no. 1-4 cross the threshold, unambiguous and specific signal are initially detectable. In the meantime, no signals are detectable of sample 5-7 because that the probe is not specific to "A" where the nucleotide is A for vaccine of SNP 36.

The LOD of this aspect could reach 10¹ copy on discriminating wild type from vaccine type. Furthermore, the specificity is relatively high because the discrimination window could reach 10⁸ copy.

### B. authentic specimen test

Four authentic specimens and two vaccine bulks (Duramune and Vanguard) are used in this aspect. The virus DNA of four authentic specimens are also isolated by using an AXYGEN®AxyPrep Body Fluid Viral DNA/RNA. The reason vaccine bulks used in this and the following aspects is to simulate the sequence type of a vaccinated dog.

Primers sequences, probe sequence, and the amplification conditions are identical to the specificity and sensitivity test. The vaccine bulks are used to carry out the amplification without dilution.

**Table 6. Conditions of the Amplification of SNP 36 for authentic specimens**

| | authentic specimen | | | | Vaccine bulks | | |
|---|---|---|---|---|---|---|---|
| Sample no. | 1 | 2 | 3 | 4 | 5 | 6 | NTC |
| DNA template | | | | | 1 µl | | |
| Primer (F) | | | | | 0.5 µM | | |
| Primer (R) | | | | | 0.5 µM | | |
| Probe | | | | | 0.2µM | | |
| dNTP | | | | | 0.25 mM | | |
| Polymerase | | | | | 5 unit | | |
| Total Volume | | | | | 20 µl | | |

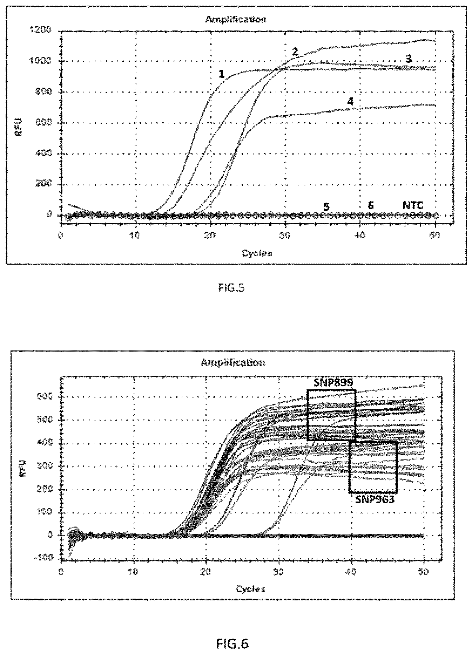

As shown in FIG. 5, four authentic specimens are detectable when their growth curve exceed the threshold. Therefore, these four authentic specimens are wild type since they contain a VP2 sequence. On the other hand, two vaccine bulks are not detectable even if their titer is high.

### ASPECT 3 Discriminating Wild Type from Vaccine Type by Using SNP 899 and SNP 963 by Taqman probe:

20 authentic specimens infected by CPV 2a, 2b, and 2c respectively and two distinct vaccine bulks (Duramune and Vanguard) are used in this aspect. The virus DNA of 20 authentic specimens are also isolated by using an AXYGEN®AxyPrep Body Fluid Viral DNA/RNA.

Primers having SEQ ID NO: 17 which comprises 30 contiguous nucleotides selected from SEQ ID: 16 for the forward primer and SEQ ID NO: 22 for the reverse primer, and probe having SEQ ID NO: 24 for SNP 899 and SEQ ID: 29 for SNP 963 were used to amplify the above-mentioned wild type samples and vaccine type samples. The primer and probe sequences are shown in Table 7. The amplification conditions are as shown is Table 8. The vaccine bulks are used to carry out the amplification without dilution.

The forward primer can be replaced to SEQ ID No: 18, 19, 20, or 21, the reverse primers can be replaced to SEQ ID No: 23, the probe for SNP 899 can be replaced to SEQ ID No: 25, 26, 27, or 28, and the probe for SNP 963 can be replaced to SEQ ID No: 30, 31, 32, or 33. The amplification conditions are identical with the aspect 1.

**Table 7. Primer and Probe Sequence**

| **Primers used in the examples** | | |
|---|---|---|
| **Sequence ID** | Function | Sequence 5'-3' |
| **SEQ ID No:17** | Forward primer | |
| **SEQ ID No:22** | Reverse primer | |
| **SEQ ID No:24** | Probe for SNP 899 | CTGAAGGAGGTACTAACTTT |
| **SEQ ID No:29** | Probe for SNP 963 | |

**Table 8. Conditions of the Amplification of SNP 899 and SNP 963**

| | authentic specimen | Vaccine bulks | |
|---|---|---|---|
| Sample no. | 1-20 | 21 | 22 |
| DNA template | 1 µl | | |
| Primer (F) | 0.5 µM | | |
| Primer (R) | 0.5 µM | | |
| SNP899 probe | 0.2µM | | |
| SNP963 probe | 0.2µM | | |
| dNTP | 0.25 mM | | |
| Polymerase | 5 unit | | |
| Total Volume | 20 µl | | |

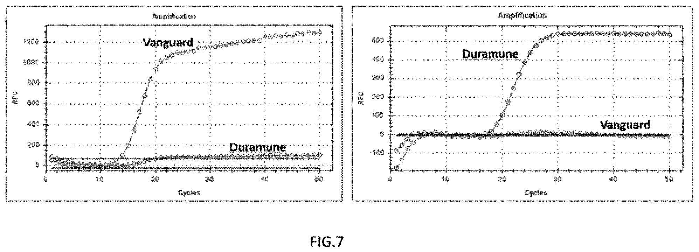

As shown in FIG. 6, both SNP 899 and SNP963 are positive on 20 authentic specimens, which means these specimens are truly infected. In contrast with FIG. 6, FIG. 7 shows that there is only one fluorescent signal would be detected if the sample was vaccinated. On the left side of FIG. 7, it shows that the SNP 963 is detectable and the SNP 899 is not detectable because the nucleotide is "T" on SNP 963 which is identical to the wild type, and the nucleotide is "C" which is not identical to the wild type. On the other hand, on the right side of FIG. 7, it shows that the SNP 899 is detectable and the SNP 963 is not detectable because the nucleotide is "G" on SNP 899 which is identical to the wild type, and the nucleotide is "A" on SNP 963, which is not identical to the wild type.

Therefore, if two distinct fluorescent signals are both detectable, it is indicative to the wild type. And if only one signal is detectable, it is indicative to the vaccine type.

### ASPECT 4 CPV 2a, 2b, and 2c detection by Immunochromatographic assay:

One CPV2a infected sample, one CPV2b infected sample, one CPV2c infected sample, and one healthy sample are used in aspect 4. The DNA was isolated by using an AXYGEN®AxyPrep Body Fluid Viral DNA/RNA. Primers having SEQ ID NO: 34 and SEQ ID NO: 3 were used to amplify a VP2 2a, 2b, and 2c sequence. The primer and probe sequences are shown in Table 2. The probe can also be replaced to SEQ ID No: 5 or SEQ ID No: 6. The labeling of primers and probe used in this aspect is different from aspect 1. The 5'-terminal of forward primer carries a DIG, and the 3'-terminal of the probe carries a FITC. The colored particle is colloidal gold.

**Table 9. Primer Sequence**

| **Primers used in the examples** | | |
|---|---|---|
| **Sequence ID** | Function | Sequence 5'-3' |
| **SEQ ID No:34** | Forward primer of VP2 | |
| **SEQ ID No:3** | Reverse primer of VP2 | |
| **SEQ ID No:4** | probe | GATTCAAAATATTAAC |

The amplification reaction is carried out which was measured and monitored in real-time on a BioRad CFX Connect Real-time System (BioRad Laboratories, Inc.). Each reaction mixture volume was 20 µl, and was amplified under the following conditions:

**Table 10. Conditions of the Amplification of the reference samples**

| | 2a | 2b | 2c | Healthy |
|---|---|---|---|---|
| DNA template | | | 1 µl | |
| Primer (F) -DIG | | | 0.3 µM | |
| Primer (R) | | | 1 µM | |
| Probe-FITC | | | 0.05 µM | |
| dNTP | | | 0.25 mM | |
| Polymerase | | | 5 unit | |
| Total Volume | 20 µl | | | |

The reaction mixtures were firstly incubated for 1 minutes at 95°C. The actual amplification reaction was carried out for 50 cycles according to the following scheme:
95 °C 1sec. → 65 °C 1 sec.

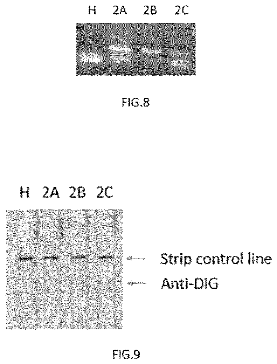

FIG.8 shows the PCR products of the corresponding region, that is VP2, by means of agarose gel. The "H" represents healthy sample. It demonstrates that the PCR reactions with the given primer do exhibit none or less cross reactivity or amplification of unspecific sequences on both wild type samples and vaccine type samples.

FIG.9 shows the lateral flow for the given pair of primers and probe. When the DIG of CPV2a, CPV2b, and CPV2c meets the anti-DIG and colloidal gold, an unambiguous and specific colored particle is initially visible. In other words, if the sample contains a VP2 sequence, a colored line can be visible on the strip. In the meantime, the colored line of healthy sample is not visible because the primer and probe are not specific to the sequence of the healthy sample.

### ASPECT 5 Discriminating Wild Type from Vaccine Type by Using SNP 36 by Immunochromatographic assay:

Three wild type specimens and three vaccine bulks are used in this aspect. The virus DNA of three authentic specimens are also isolated by using an AXYGEN®AxyPrep Body Fluid Viral DNA/RNA. The reason vaccine bulks used in this and the following aspects is to simulate the sequence type of a vaccinated dog. The labeling of primers and probe used in this aspect is different from aspect 2. The 5'-terminal of forward primer carries a DIG, and the 3'-terminal of the probe carries a FITC. The colored particle is colloidal gold. Primers sequences, probe sequence, and the amplification conditions are as below listed.

**Table 11. Primer Sequence**

| **Primers used in the examples** | | |
|---|---|---|
| **Sequence ID** | Function | Sequence 5'-3' |
| **SEQ ID No:8** | Forward primer of SNP | |
| | 36 | |
| **SEQ ID No:11** | Reverse primer of SNP 36 | |
| **SEQ ID No:13** | probe | CAGCAGGCTGACCACC |

PNA is added to the amplification to increase the specificity. The vaccine bulks are used to carry out the amplification without dilution.

**Table 12. Conditions of the Amplification of SNP 36 for authentic specimens**

| | authentic specimen | | | Vaccine bulks | | |
|---|---|---|---|---|---|---|
| Sample no. | 1 | 3 | 5 | 2 | 4 | 6 |
| DNA template | | | | 1 µl | | |
| Primer (F) -DIG | | | | 0.3 µM | | |
| Primer (R) | | | | 1 µM | | |
| PNA | | | | 0.15µM | | |
| Probe-FITC | | | | 0.05µM | | |
| dNTP | | | | 0.25 mM | | |
| Polymerase | | | | 5 unit | | |
| Total Volume | | | | 20 µl | | |

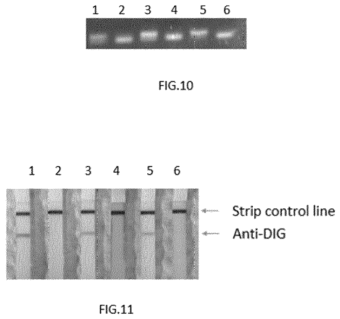

FIG.10 shows the PCR products of the corresponding region, that is SNP 36 of VP2, by means of agarose gel. Sample 1, 3, 5 are authentic specimens, and sample 2, 4, 6 are from vaccine bulks. It demonstrates that the PCR reactions with the given primer do exhibit none or less cross reactivity or amplification of unspecific sequences on both wild type samples and vaccine type samples.

FIG. 11 shows the lateral flow for the given pair of primers and probe. As above-mentioned, the primers and probe are specific to wild type of SNP 36. Thus, when the DIG of sample 1, 3, 5 meets the anti-DIG and colloidal gold, an unambiguous and specific colored particle is initially visible. In other words, if the sample contains a SNP 36 wild type sequence, a colored line can be visible on the strip. In the meantime, the colored line of vaccine bulk, that is sample 2, 4, 6, is not visible because the primers and probe are not specific to the sequence.

## Claims

1. A method of discriminating a target nucleic acid of wild type from vaccine type in a sample, comprising:
(a) providing the sample suspected to contain the target nucleic acid; and
(b)providing a pair of primers comprising a first and a second primer **characterised in that** the first primer consists of at least contiguous 12 nucleotides of a nucleic acid sequence selected from the nucleic acid sequence SEQ ID:7, and **characterised in that** the second primer consists of at least contiguous 12 nucleotides selected from the complementary nucleic acid sequences SEQ ID NO:11,12, or 13;
(c) amplifying the target nucleic acid with a template-dependent polymerase;
(d)annealing a SNP probe to the target nucleic acid to form a hybridized product during step (c) **characterised in that** the SNP probe is specific to SNP 36 of the VP2 gene of CPV 2a, 2b, or 2c, and **characterised in that** the SNP 36 probe sequence is SEQ ID NO:14 or 15, wherein the VP 2 gene is according to SEQ ID NO:1; and
(e) detecting signals generating from the hybridized product as an indicator of the presence of the target nucleic acid, wherein the presence of the signals is indicative of wild type, and the vaccine type is the sample once vaccinated with one of the vaccine selected from Duramune MX5, Canivac 5, Vanguarad plus 5 L4 CV, Nobivac Puppy DP, Canine 6II-SL, Eurican5, Virbagen DA2Parv.

2. The method according to claim 1, **characterised in that** the first primer is selected from the group consisting of SEQ ID NO: 8 to 10.

3. The method according to claim 1, **characterised in that** the signals generating from the hybridized product are fluorescent signals.

4. The method according to claim 3, **characterised in that** the 5'-terminal of the probe carries a fluorescent reporter which is selected from the group comprising FAM, HEX, VIC, CY5, or TET, and the 3'-terminal of the probe carries a quencher which is selected from a group comprising TMARA, MGB, or BHQ.

5. The method according to claim 1, **characterised in that** the signals generating from the hybridized product are analytes which are observed visually by the colored particles after a lateral flow immunochromatographic assay.

6. The method according to claim 5, **characterised in that** the 5'-terminal of either the first or second primer carries an antigen which is selected from a group comprising FITC, DIG, Biotin, Texas-red and Tamra, and the 3'-terminal of the probe carries a distinct antigen from either the first or second primer which is selected from the group comprising FITC, DIG, Biotin, Texas-red and Tamra.

7. A method of discriminating a target nucleic acid of wild type from vaccine type in a sample, comprising:
(f) providing the sample suspected to contain VP2; and
(g)providing a pair of primers comprising a first and a second primer **characterised in that** the first primer consists of at least contiguous 12 nucleotides of a nucleic acid sequence selected from the nucleic acid sequence SEQ ID:16, and **characterised in that** the second primer consists of at least contiguous 12 nucleotides selected from the complementary nucleic acid sequences SEQ ID NO: 22 or 23;
(h)amplifying the sample with a template-dependent polymerase;
(i) annealing both P1 and a P2 probes to [[a]]the target nucleic acid to form a hybridized product during step (h) **characterised in that** the P1 probe is specific to SNP 899 of the VP2 gene of CPV 2a, 2b, or 2c and the P2 probe is specific to SNP 963 of the VP2 gene of CPV 2a, 2b, or 2c, **characterised in that** the P1 is selected from a group consisting of SEQ ID NO: 24 to 27, and the P2 is selected from a group consisting of SEQ ID NO:29 to 33, wherein the VP 2 gene is according to SEQ ID NO:1; and
(j) detecting two distinct fluorescent signals of the P1 and P2 probes which are generating from the hybridized product, wherein the presence of both distinct fluorescent signals is indicative of the wild type, the presence of any one of the distinct fluorescent signals is indicative of the vaccine type, and the vaccine type is that the sample once vaccinated with one of the vaccine selected from Duramune MX5, Canivac 5, Vanguarad plus 5 L4 CV, Nobivac Puppy DP, Canine 6II-SL, Eurican5, Virbagen DA2Parvo.

8. The method according to claim 7, **characterised in that** the first primer is selected from the group consisting of SEQ ID NO: 17 to 21.

9. The method according to claim 8, **characterised in that** both the 5'-terminal of the P1 and P2 carry fluorescent reporters which are selected distinct from the group comprising FAM, HEX, VIC, CY5, or TET, and both the 3'-terminal of the probes carry quenchers which are selected from a group comprising TMARA, MGB, or BHQ, and **characterised in that** the fluorescent reporters of P1 probe and P2 probe are distinct.

## Patentansprüche

1. Verfahren zum Unterscheiden einer Zielnukleinsäure vom Wildtyp und vom Impftyp in einer Probe, welches umfasst:
(a) Bereitstellen der Probe, die mutmaßlich die Zielnukleinsäure enthält; und
(b) Bereitstellen eines Primerpaares, welches einen ersten und einen zweiten Primer umfasst, **dadurch gekennzeichnet, dass** der erste Primer aus mindestens 12 fortlaufenden Nukleotiden einer Nukleinsäuresequenz besteht, ausgewählt unter der Nukleinsäuresequenz SEQ ID:7, und **dadurch gekennzeichnet, dass** der zweite Primer aus mindestens 12 fortlaufenden Nukleotiden besteht, ausgewählt unter den komplementären Nukleinsäuresequenzen SEQ ID NR. 11, 12, oder 13;
(c) Vervielfältigen der Zielnukleinsäure mit einer Template-abhängigen Polymerase;
(d) Annealen einer SNP-Sonde an die Zielnukleinsäure, um während des Schrittes (c) ein hybridisiertes Produkt auszubilden, **dadurch gekennzeichnet, dass** die SNP-Sonde für SNP 36 des VP2-Gens von CPV 2a, 2b, oder 2c spezifisch ist, und **dadurch gekennzeichnet, dass** die SNP 36-Sondensequenz SEQ ID NR. 14 oder 15 ist, worin das VP2-Gen SEQ ID NR.1 entspricht; und
(e) Erfassen von Signalen, die von dem hybridisierten Produkt erzeugt wurden, als ein Indikator des Vorliegens der Zielnukleinsäure, wobei das Vorliegen der Signale den Wildtyp anzeigt, und der Impftyp die Probe ist, die einmal mit einem der Impfstoffe geimpft wurde, ausgewählt unter Duramune MX5, Canivac 5, Vanguard plus 5 L4 CV, Nobivac Puppy DP, Canine 6II-SL, Eurican5, Virbagen DA2Parv.

2. Verfahren nach Anspruch 1, **dadurch gekennzeichnet, dass** der erste Primer ausgewählt ist aus der Gruppe bestehend aus SEQ ID NR. 8 bis 10.

3. Verfahren nach Anspruch 1, **dadurch gekennzeichnet, dass** die von dem hybridisierten Produkt erzeugten Signale Fluoreszenzsignale sind.

4. Verfahren nach Anspruch 3, **dadurch gekennzeichnet, dass** der 5'-Terminus der Sonde einen Fluoreszenzreporter trägt, der ausgewählt ist aus der Gruppe bestehend aus FAM, HEX, VIC, CY5, oder TET, und der 3'-Terminus der Sonde einen Quencher trägt, der ausgewählt ist aus der Gruppe bestehend aus TMARA, MGB, oder BHQ.

5. Verfahren nach Anspruch 1, **dadurch gekennzeichnet, dass** die von dem hybridisierten Produkt erzeugten Signale Analyten sind, die durch die Farbpartikel nach einem seitlichen immunochromatographischen Flow-Assay visuell erfasst werden.

6. Verfahren nach Anspruch 5, **dadurch gekennzeichnet, dass** der 5'-Terminus entweder des ersten oder zweiten Primers ein Antigen trägt, das ausgewählt ist aus einer Gruppe bestehend aus FITC, DIG, Biotin, Texas-Red und Tamra, und der 3'-Terminus der Sonde ein entweder von dem ersten oder zweiten Primer verschiedenes Antigen trägt, das ausgewählt ist aus der Gruppe bestehend aus FITC, DIG, Biotin, Texas-Red und Tamra.

7. Verfahren zum Unterscheiden einer Zielnukleinsäure vom Wildtyp und vom Impftyp in einer Probe, welches umfasst:
(f) Bereitstellen der Probe, die mutmaßlich VP2 enthält; und
(g) Bereitstellen eines Primerpaares, welches einen ersten und einen zweiten Primer umfasst, **dadurch gekennzeichnet, dass** der erste Primer aus mindestens 12 fortlaufenden Nukleotiden einer Nukleinsäuresequenz besteht, ausgewählt unter der Nukleinsäuresequenz SEQ ID:16, und **dadurch gekennzeichnet, dass** der zweite Primer aus mindestens 12 fortlaufenden Nukleotiden besteht, ausgewählt unter den komplementären Nukleinsäuresequenzen SEQ ID NR. 22 oder 23;
(h) Vervielfältigen der Probe mit einer Template-abhängigen Polymerase;
(i) Annealen sowohl einer P1 als auch einer P2-Sonde an [[eine]] die Zielnukleinsäure, um während Schritt (h) ein hybridisiertes Produkt auszubilden, **dadurch gekennzeichnet, dass** die PI-Sonde für SNP 899 des VP2-Gens von CPV 2a, 2b, oder 2c spezifisch ist und die P2-Sonde für SNP 963 des VP2-Gens von CPV 2a, 2b, oder 2c spezifisch ist, **dadurch gekennzeichnet, dass** der P1 ausgewählt ist aus einer Gruppe bestehend aus SEQ ID NR. 24 bis 27, und der P2 ausgewählt ist, aus einer Gruppe bestehend aus SEQ ID NR.29 bis 33, worin das VP2-Gen der SEQ ID NR.1 entspricht; und
(j) Erfassen zweier verschiedener Fluoreszenzsignale der P1- und P2-Sonde, die von dem hybridisierten Produkt erzeugt werden, wobei das Vorliegen beider verschiedener Fluoreszenzsignale den Wildtyp anzeigt, das Vorliegen eines der verschiedenen Fluoreszenzsignale den Impftyp anzeigt, und der Impftyp die Probe ist, die einmal mit einem der Impfstoffe geimpft wurde, ausgewählt unter Duramune MX5, Canivac 5, Vanguard plus 5 L4 CV, Nobivac Puppy DP, Canine 6II-SL, Eurican5, Virbagen DA2Parvo.

8. Verfahren nach Anspruch 7, **dadurch gekennzeichnet, dass** der erste Primer ausgewählt ist aus der Gruppe bestehend aus SEQ ID NR. 17 bis 21.

9. Verfahren nach Anspruch 8, **dadurch gekennzeichnet, dass** sowohl der 5'-Terminus von P1 als auch P2 Fluoreszenzreporter tragen, die verschieden ausgewählt sind von der Gruppe bestehend aus FAM, HEX, VIC, CY5, oder TET, und beide 3'-Termini der Sonden Quencher tragen, die ausgewählt sind aus einer Gruppe bestehend aus TMARA, MGB, oder BHQ, und **dadurch gekennzeichnet, dass** die Fluoreszenzreporter der PI-Sonde und P2-Sonde verschieden sind.

## Revendications

1. Procédé de discrimination entre un acide nucléique cible de type sauvage et de type vaccin dans un échantillon, comprenant :
(a) l'utilisation de l'échantillon suspecté de contenir l'acide nucléique cible ; et
(b) l'utilisation d'une paire d'amorces comprenant une première et une deuxième amorce, **caractérisée en ce que** la première amorce est constituée par au moins 12 nucléotides contigus d'une séquence d'acide nucléique choisie parmi la séquence d'acide nucléique SEQ ID : 7 et **caractérisée en ce que** la deuxième amorce est constituée par au moins 12 nucléotides contigus choisis parmi les séquences d'acide nucléique complémentaires SEQ ID NO : 11, 12 ou 13 ;
(c) l'amplification de l'acide nucléique cible par une polymérase dépendant de la matrice ;
(d) l'hybridation d'une sonde SNP à l'acide nucléique cible pour former un produit hybridé au cours de l'étape (c), **caractérisée en ce que** la sonde SNP est spécifique au SNP 36 du gène VP2 du CPV 2a, 2b ou 2c et **caractérisée en ce que** la séquence de sonde SNP 36 est la séquence SEQ ID NO : 14 ou 15, le gène VP2 étant selon la séquence SEQ ID NO : 1 ; et
(e) la détection des signaux générés à partir du produit hybridé en tant qu'indicateur de la présence de l'acide nucléique cible, la présence des signaux étant indicatrice du type sauvage et le type vaccin étant l'échantillon une fois vacciné avec l'un des vaccins choisis parmi Duramune MX5, Canivac 5, Vanguarad Plus 5 L4 CV, Nobivac Puppy DP, Canine 6II-SL, Eurican5, Virbagen DA2 Parvo.

2. Procédé selon la revendication 1, **caractérisé en ce que** la première amorce est choisie dans le groupe constitué par les séquences SEQ ID NO : 8 à 10.

3. Procédé selon la revendication 1, **caractérisé en ce que** les signaux générés à partir du produit hybridé sont des signaux fluorescents.

4. Procédé selon la revendication 3, **caractérisé en ce que** l'extrémité 5' de la sonde porte un rapporteur fluorescent qui est choisi dans le groupe comprenant FAM, HEX, VIC, CY5 ou TET et l'extrémité 3' de la sonde porte un agent d'extinction qui est choisi dans un groupe comprenant TMARA, MGB ou BHQ.

5. Procédé selon la revendication 1, **caractérisé en ce que** les signaux générés à partir du produit hybridé sont des analytes qui sont observés visuellement par les particules colorées après un dosage immunochromatographique à écoulement latéral.

6. Procédé selon la revendication 5, **caractérisé en ce que** l'extrémité 5' de la première ou de la deuxième amorce porte un antigène qui est choisi dans un groupe comprenant FITC, DIG, biotine, Texas-rouge et Tamra et l'extrémité 3' de la sonde porte un antigène distinct de l'un ou de l'autre antigène de la première ou de la deuxième amorce qui est choisi dans le groupe comprenant FITC, DIG, biotine, Texas-rouge et Tamra.

7. Procédé de discrimination entre un acide nucléique cible de type sauvage et de type vaccin dans un échantillon, comprenant :
(f) l'utilisation de l'échantillon suspecté de contenir VP2 ; et
(g) l'utilisation d'une paire d'amorces comprenant une première et une deuxième amorce, **caractérisée en ce que** la première amorce est constituée par au moins 12 nucléotides contigus d'une séquence d'acide nucléique choisie parmi la séquence d'acide nucléique SEQ ID : 16 et **caractérisée en ce que** la deuxième amorce est constituée par au moins 12 nucléotides contigus choisis parmi les séquences d'acide nucléique complémentaires SEQ ID NO : 22 ou 23 ;
(h) l'amplification de l'échantillon par une polymérase dépendant de la matrice ;
(i) l'hybridation des deux sondes P1 et P2 à [[a]] l'acide nucléique cible pour former un produit hybridé pendant l'étape (h), **caractérisée en ce que** la sonde P1 est spécifique au SNP 899 du gène VP2 du CPV 2a, 2b ou 2c et la sonde P2 est spécifique au SNP 963 du gène VP2 du CPV 2a, 2b ou 2c, **caractérisée en ce que** P1 est choisie dans un groupe constitué par les séquences SEQ ID NO : 24 à 27 et P2 est choisie dans un groupe constitué par les séquences SEQ ID NO : 29 à 33, le gène VP2 étant selon SEQ ID NO : 1 ; et
(j) la détection de deux signaux fluorescents distincts des sondes P1 et P2 qui sont générés à partir du produit hybridé, la présence des deux signaux fluorescents distincts étant indicatrice du type sauvage, la présence de l'un quelconque des signaux fluorescents distincts étant indicatrice du type vaccin et le type vaccin étant l'échantillon une fois vacciné avec l'un des vaccins choisis parmi Duramune MX5, Canivac 5, Vanguarad Plus 5 L4 CV, Nobivac Puppy DP, Canine 6II-SL, Eurican5, Virbagen DA2 Parvo.

8. Procédé selon la revendication 7, **caractérisé en ce que** la première amorce est choisie dans le groupe constitué par les séquences SEQ ID NO : 17 à 21.

9. Procédé selon la revendication 8, **caractérisé en ce que** les deux extrémités 5' de P1 et de P2 portent des rapporteurs fluorescents qui sont choisis de manière distincte dans le groupe comprenant FAM, HEX, VIC, CY5 ou TET et les deux extrémités 3' des sondes portent des agents d'extinction qui sont choisis dans un groupe comprenant TMARA, MGB ou BHQ et **caractérisé en ce que** les rapporteurs fluorescents de la sonde P1 et P2 sont distincts.
